# EUROPEAN PATENT APPLICATION

(11) **EP 4 353 201 A1**
(43) Date of publication of application: **17.04.2024**
(21) Application number: 21944438.7
(22) Date of filing: 08.06.2021
(51) Int. Cl.: A61F 2/915

(54) **STENT**

(71) Applicant: Global Vascular Co., Ltd., Tokyo 162-0843 (JP)
(72) Inventor: KAMEI, Shunsuke, Tokyo 167-0033 (JP); MAEGAWA, Shunto, Tokyo 103-0028 (JP); BITO, Kenta, Tokyo 103-0028 (JP); HASEBE, Terumitsu, Kawasaki-shi, Kanagawa 215-0018 (JP)
(74) Representative: Berggren Oy
(86) International application number: PCT/JP2021/021792
(87) International publication number: WO 2022/259382

(57) **Abstract**

The disclosure provides a stent including: an annular first strut forming a wave shape; an annular second strut that is adjacent to the first strut in the axial direction and forms a wave shape; a first extension portion that is formed on a first crest as some of plural crests protruding toward the second strut in the first strut and extends from both sides with respect to a bisector of the first crest toward the second strut; a second extension portion that is formed on a second crest, which is located to be shifted in a circumferential direction with respect to the first crest among plural crests protruding toward the first strut side in the second strut, and extends from both sides with respect to a bisector of the second crest toward the first strut; and a bridge portion bridged between the first extension portion and the second extension portion.

## Description

### Technical Field

The present disclosure relates to a stent.

### Background Art

As an aspect of medical stents, for example, U.S. Patent No. 7264633 discloses a stent having a link connecting crests facing each other in two adjacent struts.

### SUMMARY OF INVENTION

### Technical Problem

The stent disclosed in U.S. Patent No. 7264633 has a configuration in which the link extends only from one side with respect to the tops of the crests of the strut. Therefore, in this stent, stress is likely to concentrate on the link and a peripheral portion of the link as the stent transitions from one of a reduced diameter state or an expanded diameter state to the other. This stress concentration causes a deterioration in shape accuracy of the stent.

An object of the disclosure is to improve shape accuracy of a stent as compared with a configuration in which a portion connecting circumferentially shifted crests of struts, adjacent in the axial direction, extends only from one side with respect to a bisector of a first crest or a second crest.

### Solution to Problem

A stent according to a first aspect includes: a first strut that is arranged along a cylindrical surface and forms a wave shape having an amplitude direction that is an axial direction of the cylindrical surface; a second strut that is arranged along the cylindrical surface, that forms a wave shape having an amplitude direction that is the axial direction, and that is adjacent to the first strut in the axial direction; a first extension portion that is formed on a first crest and extends along a first bisector, bisecting the first crest, from both sides with respect to the first bisector toward the second strut, the first crest being at least one of a plurality of crests in the first strut protruding toward the second strut; a second extension portion that is formed on a second crest and extends along a second bisector, bisecting the second crest, from both sides with respect to the second bisector toward the first strut, the second crest being displaced in a circumferential direction with respect to the first crest among a plurality of crests in the second strut protruding toward the first strut side; and a bridge portion extending from the first extension portion to the second extension portion.

A stent according to a second aspect is the stent according to the first aspect, in which a thickness of the bridge portion is equal to and uniform with a thickness of the first extension portion or the second extension portion.

A stent according to a third aspect is the stent according to the first or second aspect, in which the first extension portion has an elliptical shape whose major axis is the first bisector, and the second extension portion has an elliptical shape whose major axis is the second bisector.

A stent according to a fourth aspect is the stent according to any one of the first to third aspects, in which an edge portion of the bridge portion has a curved shape in which one end on a side closer to the first extension portion forms a common tangent with an edge of the first extension portion at a boundary with respect to the first extension portion and the other end on a side closer to the second extension portion forms a common tangent with an edge of the second extension portion at a boundary with respect to the second extension portion when viewed along the radial direction of the cylindrical surface.

A stent according to a fifth aspect is the stent according to any one of the first to fourth aspects, including a shape memory alloy.

A stent according to a sixth aspect is the stent according to any one of the first to fifth aspects, further including a coating layer on the surface.

A stent according to a seventh aspect is the stent according to the sixth aspect, in which the coating layer includes a ceramic layer.

A stent according to an eighth aspect is the stent according to the sixth or seventh aspect, in which the coating layer includes a drug-eluting coating layer.

A stent according to a ninth aspect is the stent according to any one of the first to eighth aspects, in which a thickness of each of the first strut and the second strut is in a range of from 0.6 times a minimum width B of each of the first strut and the second strut in a direction along the cylindrical surface to B.

### Advantageous Effects of Invention

With the stent according to the first aspect, shape accuracy of the stent is improved as compared with a configuration in which a portion connecting the circumferentially shifted crests of the struts, adjacent in the axial direction, extends only from one side with respect to the bisector of the first crest or the second crest.

With the stent according to the second aspect, the shape accuracy of the stent is improved as compared with a configuration in which the thickness of the bridge portion is smaller than the thickness of the first extension portion or the second extension portion.

With the stent according to the third aspect, the shape accuracy of the stent is improved as compared with a configuration in which the first extension portion or the second extension portion has a rectangular shape when viewed from the radial direction.

With the stent according to the fourth aspect, the shape accuracy of the stent is improved as compared with a configuration in which the ends of the edge portion of the bridge portion are linear.

With the stent according to the fifth aspect, in a case in which the stent is made of the shape memory alloy, the shape accuracy of the stent is improved as compared with a configuration in which the portion connecting the first crest and the second crest extends only from one side with respect to the bisector of the first crest or the second crest.

With the stent according to the sixth aspect, in a configuration in which the coating layer is provided, the coating layer is less likely to be peeled off as compared with the configuration in which the portion connecting the first crest and the second crest extends only from one side with respect to the bisector of the first crest or the second crest.

With the stent according to the seventh aspect, in a configuration in which the coating layer includes the ceramic layer, a deterioration of the stent over time is suppressed as compared with the configuration in which the portion connecting the first crest and the second crest extends only from one side in the circumferential direction with respect to the bisector of the first crest or the second crest.

With the stent according to the eighth aspect, in a configuration in which the coating layer includes the drug-eluting coating layer, a drug elution effect of the stent is maintained for a long period of time as compared with the configuration in which the portion connecting the first crest and the second crest extends only from one side with respect to the bisector of the first crest or the second crest.

With the stent according to the ninth aspect, the stent has a larger inner diameter as compared with a configuration in which the thickness of the strut exceeds the minimum width of the strut. In addition, with the stent according to the ninth aspect, the stent is less likely to be distorted as compared with a configuration in which the thickness of the strut is less than 0.6 times the minimum width of the strut.

### BRIEF DESCRIPTION OF DRAWINGS

Fig. 1 is a developed view in which a stent in a processed state according to an embodiment of the disclosure is developed in a planar shape along the circumferential direction.
Fig. 2 is an enlarged view illustrating a link of the stent according to the embodiment of the disclosure.
Fig. 3 is a cross-sectional view illustrating a substrate layer and a coating layer which form the stent according to the embodiment of the disclosure.
Fig. 4 is an enlarged view illustrating a link of a stent according to a comparative embodiment of the disclosure.
Fig. 5 is a developed view in which a stent according to a modification of the embodiment of the disclosure is developed in the circumferential direction.
Fig. 6 is an enlarged view illustrating a link of the stent according to the modification of the embodiment of the disclosure.
Fig. 7 is a perspective view of the stent in an expanded diameter state according to the embodiment of the disclosure.
Fig. 8 is a perspective view of the stent in a reduced diameter state according to the embodiment of the disclosure.
Fig. 9 is a developed view in which a stent according to a modification of the embodiment of the disclosure is developed in the circumferential direction.

### DESCRIPTION OF EMBODIMENTS

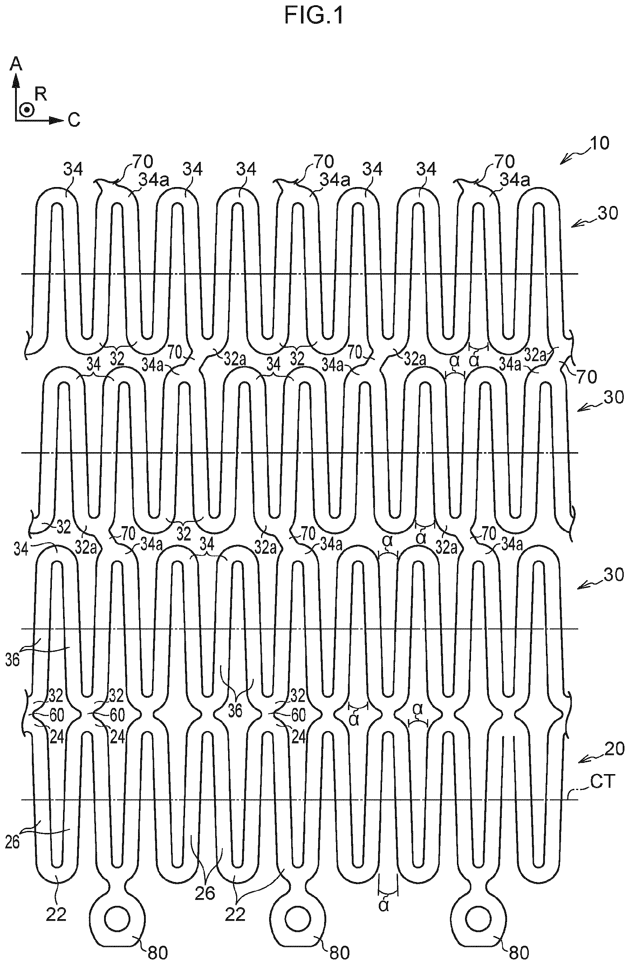

Examples of a stent according to an embodiment of the disclosure will be described with reference to Figs. 1 to 9.

### (Stent 10)

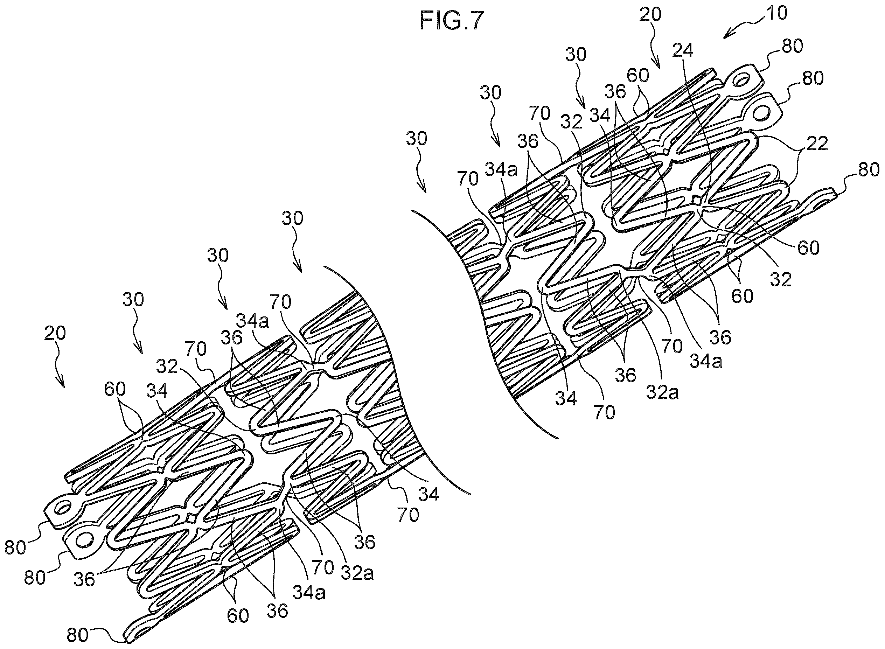

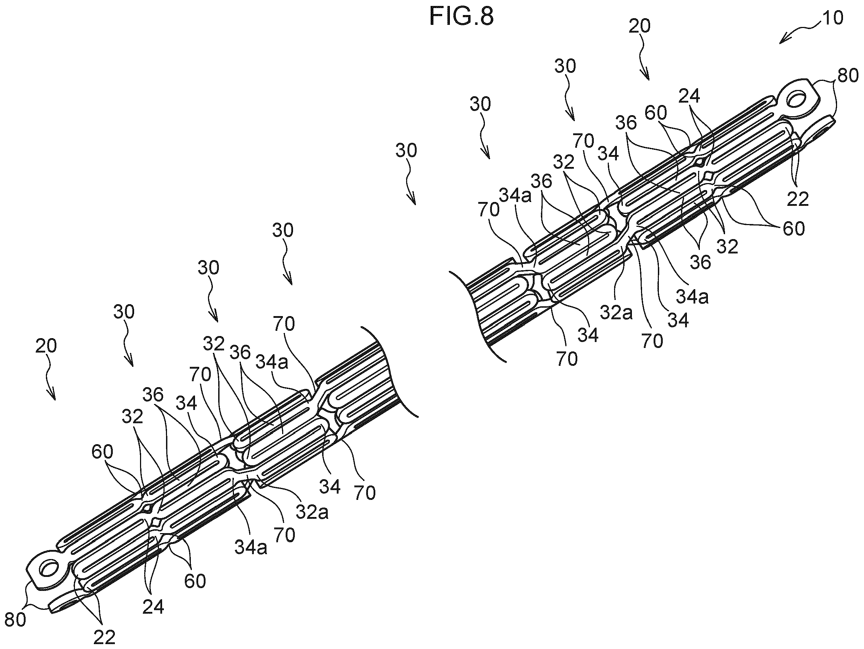

A stent 10 according to the present embodiment is a substantially cylindrical (see Figs. 7 and 8) stent used to expand a lumen of a stenosed part in a case in which stenosis or the like occurs in a biological organ having a lumen structure such as a blood vessel. Specifically, the stent 10 is formed along a cylindrical surface, which has a length L and a diameter D, of a tubular member (details of which will be described later) that is a material of the stent 10. The term "cylindrical surface" used in the following description includes, in addition to the cylindrical surface having the length L and the diameter D, a surface as a part of the cylindrical surface, a surface obtained by extending the cylindrical surface in the axial direction, and a surface obtained by expanding and reducing of the cylindrical surface in diameter. Fig. 1 is a developed view in which the stent 10 in a processed state cut out from the tubular member by laser processing is developed in a planar shape along the circumferential direction. That is, the diameter of the stent 10 illustrated in Fig. 1 is D. The stent 10 is, for example, a self-expanding stent, and has a function of being able to switch between an expanded diameter state illustrated in Fig. 7 and a reduced diameter state illustrated in Fig. 8. A diameter of the stent 10 in the expanded diameter state is larger than the diameter D of the stent in the processed state. In addition, a diameter of the stent 10 in the reduced diameter state is smaller than the diameter D of the stent 10 in the processed state. The stent according to the disclosure is not limited to the self-expanding stent, and may be a balloon expanding stent. In the following description, a dimensional shape of the stent 10 in the processed state will be described unless otherwise specified.

Note that arrows A, R, and C in each drawing respectively indicate the axial direction, the radial direction, and the circumferential direction of the stent 10.

The stent 10 has end struts 20 arranged at both ends in the axial direction of the stent 10 and a plurality of struts 30 arranged side by side in the axial direction between the two end struts 20 as illustrated in a developed manner on a plane in Fig. 1. The stent 10 further includes links 60 connecting the end strut 20 and the strut 30, and links 70 connecting the adjacent struts 30. The stent 10 further includes markers 80 each projecting outward in the axial direction from a part of the end strut 20. In the stent 10, the end struts 20, the struts 30, the links 60 and 70, and the markers 80 are integrally formed.

The end strut 20 is an annular member along the cylindrical surface. The end strut 20 is formed in a wave shape whose amplitude direction is the axial direction of the stent 10.

The end strut 20 has a plurality of crests 22 protruding toward a side opposite to the strut 30 adjacent to the end strut 20 with respect to a center line CT of the wave of the end strut 20. The end strut 20 further has a plurality of crests 24 projecting toward the strut 30 adjacent to the end strut 20 with respect to the center line CT of the wave of the end strut 20. The end strut 20 further has a straight portion 26 linearly connecting the crest 22 and the crest 24. At a boundary between the straight portion 26 and each of the crest 22 and the crest 24, an edge of the straight portion 26 forms a common tangent of an edge of the crest 22 and an edge of the crest 24. Each of the crests 22 and 24 of the end strut 20 according to the disclosure indicates a folded portion of the wave of the end strut 20. In the embodiment, each of the crest 22 and the crest 24 is formed in an arc shape in both the inner peripheral edge and the outer peripheral edge when viewed from the radial direction.

As illustrated in a developed manner on the plane in Fig. 1, the markers 80 are substantially disk-shaped members expanding along the cylindrical surface and formed so as to project from the tops of some crests 22 of the plurality of crests 22 of the end struts 20 to a side opposite to the crests 22 with respect to the crests 24.

### (Strut 30)

The strut 30 is an annular member arranged along the cylindrical surface between the two end struts 20. The plurality of struts 30 are arranged side by side at intervals fixed in the axial direction. Each of the struts 30 is formed in a wave shape whose amplitude direction is the axial direction of the stent 10. Each of the struts 30 has the same shape. In the embodiment, each of the struts 30 has substantially the same shape as the end strut 20. More specifically, the strut 30 of the embodiment has a wave shape having nine cycles in one round along the cylindrical surface, and has crests 32, protruding toward one side in the axial direction with respect to the center line of the wave, and crests 34 protruding toward the other side. That is, each of the struts 30 of the embodiment has nine crests 32 and nine crests 34. The strut 30 further has a straight portion 36 linearly connecting the crest 32 and the crest 34. At a boundary between the straight portion 36 and each of the crest 32 and the crest 34, an edge of the straight portion 36 forms a common tangent of an edge of the crest 32 and an edge of the crest 34. Each of the crests 32 and 34 of the strut 30 according to the disclosure indicates a folded portion of the wave of the strut 30.

In the embodiment, each of the crest 32 and the crest 34 is formed in an arc shape in both the inner peripheral edge and the outer peripheral edge when viewed from the radial direction. In addition, bisectors (including a bisector D1 to be described later) that bisect the crest 32 and the crest 34 when viewed from the radial direction are parallel to the axial direction. In other words, a pair of the straight portions 36 connected to one crest 32 or 34 is formed symmetrically (that is, equiangularly) with respect to the bisector. Furthermore, an interval between a pair of the straight portions 36 connected to one crest 32 gradually increases toward the crests 34 adjacent in the circumferential direction. In addition, an interval between a pair of the straight portions 36 connected to one crest 34 gradually widens toward the crests 32 adjacent in the circumferential direction. An angle α formed between a pair of the straight portions 36 is preferably in a range of from 25° to 50° in the stent 10 in the expanded diameter state.

In the stent 10 in the reduced diameter state, the angle α formed between a pair of the straight portions 36 is preferably in a range of from -10° to -5°. When the angle α formed between a pair of straight portions 36 connected to one crest 32 has a negative value, an interval between the pair of straight portions 36 gradually narrows toward the crests 34 adjacent in the circumferential direction as illustrated in Fig. 8. When the angle α formed between a pair of straight portions 36 connected to one crest 34 has a negative value, an interval between the pair of straight portions 36 gradually narrows toward the crests 32 adjacent in the circumferential direction as illustrated in Fig. 8.

As illustrated in Fig. 1, the plurality of struts 30 are arranged side by side in the axial direction. In addition, two struts 30 adjacent to each other in the axial direction are arranged such that phases of waves in the circumferential direction are shifted within a range of from 10° to 90°. The amount of the shift between the phases of the waves of the two adjacent struts 30 is preferably within a range of from 5° to 20°.

In addition, the nth (where n is an integer of one or more) strut and the (n - 2)th or (n + 2)th strut among the plurality of struts 30 arrayed side by side in the axial direction are arranged such that phases of waves in the circumferential direction are the same.

The strut 30 adjacent to the end strut 20 is arranged such that a phase of a wave in the circumferential direction is shifted by 180° with respect to a phase of a wave in the circumferential direction of the end strut 20. That is, the strut 30 adjacent to one end strut 20 (the end strut 20 on the upper side on the paper surface in Fig. 1) is arranged such that the crest 32 faces the crest 24 of the end strut 20 in the axial direction. In addition, the strut 30 adjacent to the other end strut 20 (the end strut 20 on the lower side on the paper surface in Fig. 1) is arranged such that the crest 34 faces the crest 24 of the end strut 20 in the axial direction. Each of the crests 24 of the struts 30 adjacent to these end struts 20 is connected to the crest 24 of the end strut 20 facing each other in the axial direction through the link 60.

In the two struts 30 adjacent to each other in the axial direction, some crests 32a of the plurality of crests 32 of one strut 30 and some crests 34a of the plurality of crests 34 of the other strut 30 are connected through the link 70 to be described in detail later. For example, in the embodiment including the struts 30 each having the wave shape of nine cycles in one round, the struts 30 adjacent in the axial direction are connected by three links 70 arranged at equal intervals in the circumferential direction. The crest 34a of the other strap 30 connected to the crest 32a of the one strut 30 by the link 70 is the crest 34 closest to the crest 32a of the one strut 30 among the plurality of crests 34 of the other strut 30.

The link 70 connecting the nth (where n is an even number of two or more) strut 30 and the (n - 1)th strut 30 among the plurality of struts 30 arrayed side by side in the axial direction is shifted in the circumferential direction with respect to the link 70 connecting the nth strut 30 and the (n + 1)th strut 30. Furthermore, the link 70 connecting the nth strut 30 and the (n - 1)th strut 30 is inclined in the opposite direction with respect to the link 70 connecting the nth strut 30 and the (n + 1)th strut 30. Here, the crest 32a is an example of a first crest, and the crest 34a is an example of a second crest. When the odd-numbered strut 30 is an example of a first strut, the even-numbered strut 30 is an example of a second strut. Alternatively, when the odd-numbered strut 30 is an example of the second strut, the even-numbered strut 30 is an example of the first strut.

### (Detailed Structure of Link 70)

In the stent 10, the link 70 connects the crest 32a of one strut 30 and the crest 34a of the other strut 30 of the two struts 30 adjacent in the axial direction as described above. The link 70 includes an extension portion 44 formed on the crest 32a, an extension portion 54 formed on the crest 34a, and a bridge portion 72 bridged between the extension portion 44 and the extension portion 54. In the following description, a bisector of the crest 32a is referred to as a bisector D1, and a bisector of the crest 34a is referred to as a bisector D2.

The extension portion 44 is a portion extending along the bisector D1 from an outer peripheral edge of the crest 32a on both sides of the bisector D1 toward the strut 30 to be connected by the link 70. In the embodiment, the extension portion 44 has a line-symmetrical shape with respect to the bisector D1. More specifically, the extension portion 44 is formed in an elliptical shape with the bisector D1 as the major axis direction. In the embodiment, the elliptical shape means that an outer edge when viewed from the radial direction forms a part of an ellipse. An elliptical edge portion of the extension portion 44 has a common tangent with the crest 32a at two boundaries with respect to the crest 32a. That is, the crest 32a and the extension portion 44 are smoothly continuous so as not to cause a step or an inflection point.

The extension portion 54 is formed in the same manner as the extension portion 44. That is, the extension portion 54 has a shape symmetrical in the axial direction with respect to the extension portion 44 although detailed description is omitted, and extends along the bisector D2 from the crest 34a toward the strut 30 to be connected by the link 70. As described above, the extension portions 44 and 54 can also be regarded as portions obtained by extending the crests 32a and 34a along the bisectors D1 and D2, respectively. Here, the extension portion 44 is an example of a first extension portion, and the extension portion 54 is an example of a second extension portion. Note that no extension portion is formed in the crests 32 and 34 other than the crests 32a and 34a. In other words, each of the crests 32a and 34a has an elliptical outer edge and an arcuate inner edge, and can also be regarded as a crest longer in the axial direction than the other crests 32 and 34.

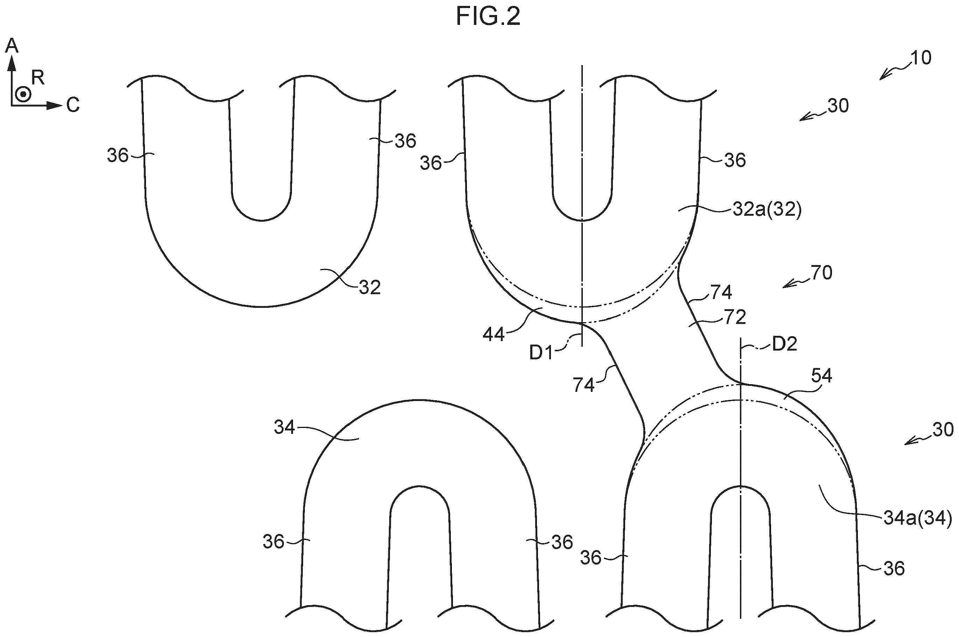

As illustrated in Fig. 2, the bridge portion 72 is a substantially quadrangular portion formed between the first extension portion 44 and the second extension portion 54 when viewed from the radial direction (that is, the R direction). The bridge portion 72 extends in a direction inclined with respect to the axial direction (that is, the bisectors D1 and D2). Specifically, the bridge portion 72 is formed along an extending direction extending from the crest 32a toward the crest 34a. In the embodiment, the bridge portion 72 has, as a center line, an imaginary straight line (not illustrated) passing through the center of the ellipse forming the outer edge of the extension portion 44 and the center of an ellipse forming an outer edge of the extension portion 54.

The bridge portion 72 has two edge portions 74 extending in a direction along the extending direction. One edge portion 74 extends from the vicinity of the top of the extension portion 44 to a portion on a side closer to the straight portion 36 between the bisectors D1 and D2 in the extension portion 54. The other edge portion 74 extends from the vicinity of the top of the extension portion 54 to a portion on a side closer to the straight portion 36 between the bisectors D1 and D2 in the extension portion 44.

The edge portion 74 of the bridge portion 72 has both ends formed in a curved shape, and a portion between the both ends is formed in a linear shape parallel to the imaginary straight line. Specifically, when viewed from the radial direction, in each of the edge portions 74, an end on a side closer to the top of the extension portion 44 or an end on a side closer to the top of the extension portion 54 forms a common tangent with the edge of the extension portion 44 or the edge of the extension portion 54 at a boundary with respect to the vicinity of the top of the extension portion 44 or the extension portion 54. In addition, when viewed from the radial direction, in each of the edge portions 74, an end on the side closer to the straight portion 36 of the extension portion 44 or an end on the side closer to the straight portion 36 of the extension portion 54 forms a common tangent with the edge of the extension portion 44 or the edge of the extension portion 54 at a boundary with respect to the portion on the side closer to the straight portion 36 of the extension portion 44 or the portion on the side closer to the straight portion 36 of the extension portion 54. That is, one end of the edge portion 74 of the bridge portion 72 on the side closer to the extension portion 44 forms the common tangent with the edge of the extension portion 44 at the boundary with respect to the extension portion 44 when viewed from the radial direction. The other end of the edge portion 74 of the bridge portion 72 on the side closer to the extension portion 54 forms the common tangent with the edge of the second extension portion 54 at the boundary with the second extension portion 54 when viewed from the radial direction.

In the stent 10, the minimum width of the bridge portion 72 (that is, a width in a direction orthogonal to the inclined direction of the bridge portion 72) is substantially equal to the minimum width of the straight portion 36 of the strut 30.

### (Cross-Sectional Structure of Stent 10)

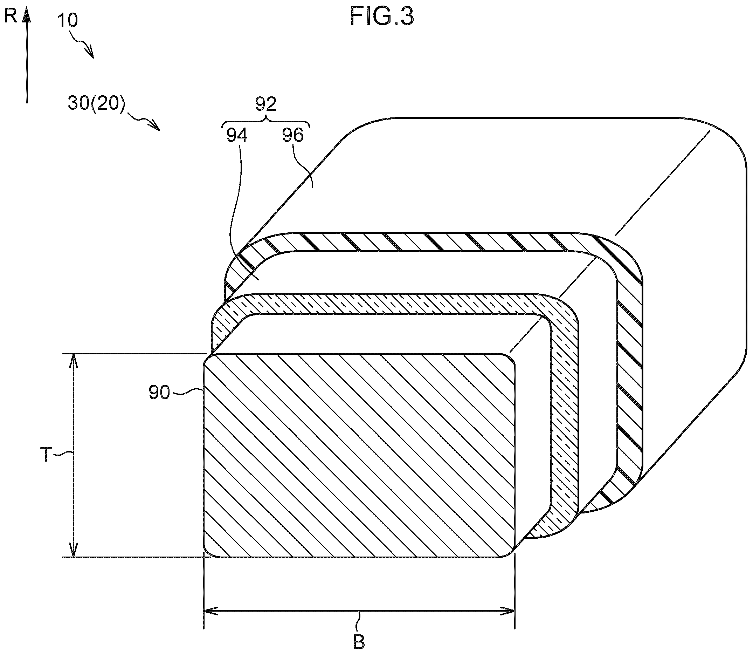

The stent 10 has a cross section formed in layers including a substrate layer 90 and a coating layer 92. Specifically, as illustrated in Fig. 3, the wave-shaped strut 30 has, in a cross section cut along an imaginary plane orthogonal to a direction along a wavy line of the strut 30, the substrate layer 90 located at the center of the cross section, and the coating layer 92 formed on the surface of the substrate layer 90. The same applies to the end strut 20, the links 60 and 70, and the marker 80.

The substrate layer 90 is made of a material containing metal having shape memory properties, such as a nickel-titanium alloy. That is, the stent 10 is made of a shape memory alloy. The stent 10 has a shape formed, for example, by cutting out a tubular member, which is made of the metal and has the cylindrical surface having the length L and the diameter D and, by laser processing. Note that the material forming the substrate layer 90 according to the disclosure is not limited to the nickel-titanium alloy, and may contain metal such as iron, copper, titanium, nickel, cobalt, chromium, aluminum, zinc, manganese, tantalum, tungsten, platinum, or gold. In particular, examples of the metal forming the substrate layer 90 preferably include at least one selected from the group consisting of titanium, nickel, cobalt, chromium, tantalum, platinum, and gold. In addition, the material forming the substrate layer 90 may contain alloys such as a cobalt-chromium alloy, a copper-aluminum-manganese alloy, a copper-zinc alloy, a nickel-aluminum alloy, and stainless steel. In particular, examples of the metal forming the substrate layer 90 preferably include a nickel-titanium alloy, a cobalt-chromium alloy, or stainless steel among the alloys.

In addition, the material forming the substrate layer 90 according to the disclosure is not limited to the metal, and may contain, for example, a polymer such as polyester or a polycarbonate copolymer. In addition, examples of the polymer forming the substrate layer 90 according to the disclosure may include a biodegradable polymer having biodegradability, such as polylactic acid, polyhydroxybutyrate, polyglycolic acid, a lactic acid-glycolic acid copolymer, polycaprolactone, a lactic acid-caprolactone copolymer, a glycolic acid-caprolactone copolymer, poly-γ-glutamic acid, or collagen.

The substrate layers 90 of the end strut 20 and the strut 30 have a rectangular cross section as illustrated in Fig. 3. A thickness of the cross section in the R direction is a thickness T. The minimum width in the width direction with respect to the thickness T of the cross section is a minimum width B. The thickness T of the substrate layer 90 of each of the end strut 20 and the strut 30 is in a range of from 0.6 times the minimum width B to the minimum width B. As a specific example, the thickness T of each of the end strut 20 and the strut 30 is 120 µm ± 10 µm, and the minimum width B of each of the end strut 20 and the strut 30 is 150 µm ± 10 µm.

Each of the end strut 20, the strut 30, the links 60 and 70, and the marker 80 is formed such that the thickness of the substrate layer 90 in the R direction is uniformly the thickness T. That is, the thickness of the bridge portion 72 in the R direction is equal to and uniform with the thicknesses of the extension portion 44 and the extension portion 54 in the R direction.

### (Coating Layer 92)

The coating layer 92 includes a diamond-like carbon layer (DLC layer) 94 formed on the surface of the substrate layer 90 and a drug-eluting coating layer 96 formed on the surface of the DLC layer 94.

### (DLC Layer 94)

The DLC layer 94 is made of diamond-like carbon containing fluorine (F) and silicon (Si). The DLC layer 94 is an example of a ceramic layer. Since the coating layer 92 includes the DLC layer 94, which is an example of the ceramic layer, in the stent 10, elution of ions of metal forming the stent is suppressed, and a biological reaction of the stent 10 with respect to a biological organ or blood is suppressed. By containing F and Si, the DLC layer 94 has followability to stably follow deformation of the stent 10 accompanying a movement of a living body, and flexibility to flexibly deform against various types of stress when following the deformation. In addition, the DLC layer 94 has antithrombogenic properties by containing F.

Note that the ceramic layer according to the disclosure is not limited to the DLC layer 94 as long as including a metal oxide layer, a metal nitride layer, a Si layer, or a carbon layer. The metal oxide layer or the metal nitride layer according to the disclosure preferably contains titanium, nickel, cobalt, chromium, aluminum, zinc, manganese, tantalum, or the like. The Si layer according to the disclosure preferably contains silicon carbide or silicon dioxide. The carbon layer according to the disclosure preferably contains diamond-like carbon or graphene.

The DLC layer 94 may be a layer formed by any method as long as the method allows the layer to contain F and Si. The DLC layer 94 can be formed using a known method such as a vapor deposition method or a sputtering method. In particular, the DLC layer 94 is preferably formed by an ion vapor deposition method. Furthermore, the DLC layer 94 is more preferably formed by a chemical vapor deposition (CVD) method or a physical vapor deposition (PVD) method. The DLC layer 94 formed by the CVD method or the PVD method is less likely to be peeled off from the stent 10 according to the disclosure as compared with a configuration formed by a method other than the CVD method or the PVD method. Details of the CVD method and the PVD method will be described below.

Examples of the CVD method include a plasma enhanced-chemical vapor deposition (PE-CVD) method and a thermal chemical vapor deposition method. A thin film containing F, Si, and C can be formed by thin film synthesis using the PE-CVD method.

The PE-CVD method is a type of chemical vapor deposition (CVD) method using a gas as a raw material, and is a method in which a raw material gas is introduced into a vacuum vessel, and plasma is generated to cause a chemical reaction, thereby depositing a film. The CVD method is a generic term for methods of depositing a film on a substrate using a chemical reaction.

As an energy source for causing the chemical reaction, heat, plasma, laser, or the like is used. In the CVD method, a gaseous body (gas) is used as a raw material. Therefore, the quality of a film that can be formed can be freely changed by selecting a raw material gas, and various elements can be added, if desired.

In the PE-CVD method, a hydrocarbon gas or a gas containing an element to be desirably added is caused to flow as a raw material gas into a vacuum vessel, and plasma is generated to cause a chemical reaction, thereby depositing a film. As power for generating the plasma, DC power or AC power such as a high frequency or microwave is suitably used. In the PE-CVD method, the use of plasma can cause a chemical species having a high degree of activation to react, and the reaction can be caused at a low temperature.

The DLC layer according to the disclosure can be formed using an inductively coupled plasma-chemical vapor deposition (ICP-CVD) apparatus using a high frequency. As the ICP-CVD apparatus, for example, YH-100NX manufactured by ONWARD GIKEN Co., Ltd. can be used.

In the ICP-CVD apparatus, plasma is generated between ring-shaped electrodes by high-frequency discharge, and a bias voltage is applied to a jig on which a substrate is placed so that ionized or excited chemical species are adsorbed to deposit and form a film. As conditions for the film formation, parameters, such as a processing time, a high-frequency output, a bias voltage, a raw material gas flow rate, a high-frequency pulse amplitude, a bias pulse amplitude, and a high-frequency voltage, a bias voltage, and a raw material gas flow rate at the time of plasma ignition, can be controlled.

Examples of the PVD method include a plasma ion implantation method, a vacuum vapor deposition method, and a sputtering method.

As the raw material, a mixed raw material obtained by mixing a silane compound and a fluorine-containing aliphatic hydrocarbon is used.

As the silane compound, an organosilicon compound containing carbon is preferable. Examples of the organosilicon compound include a compound represented by the following Formula S.

SiRₓH₄₋ₓ: Formula S

In Formula S, R represents an alkyl group having 1 to 4 carbon atoms, and x represents an integer of 1 to 4.

Examples of the compound represented by Formula S include tetramethylsilane, tetraethylsilane, trimethylsilane, diethylsilane, methyldiethylsilane, and diethyldimethylsilane.

As the fluorine-containing aliphatic hydrocarbon, a fluorine-containing aliphatic hydrocarbon having 1 to 4 carbon atoms is preferable, and a perfluoro hydrocarbon having 1 to 4 carbon atoms is more preferable. Examples of the fluorine-containing aliphatic hydrocarbon include tetrafluoromethane (CF₄), hexafluoroethane (C₂F₆), octafluoropropane, and perfluorobutane (C₄F₁₀).

As the raw material, hydrocarbon can be further used.

In the case of the CVD method, a saturated hydrocarbon (for example, methane (CH₄) or ethane (C₂H₆)), an unsaturated hydrocarbon (for example, acetylene (C₂H₂) or benzene (C₆H₆)), or the like can be used as the hydrocarbon.

In the case of the PVD method, solid carbon can be used as the hydrocarbon.

In a DLC layer formation step, a silane compound is vaporized, and the vaporized silane compound and a fluorine-containing aliphatic hydrocarbon (an unsaturated hydrocarbon may be added) are introduced into a chamber to form a film. At this time, it is preferable to control and gradually change partial pressures of the silane compound and the fluorine-containing aliphatic hydrocarbon. A raw material in which the silane compound and the fluorine-containing aliphatic hydrocarbon are mixed can form the DLC layer according to the disclosure by mixing the silane compound and the fluorine-containing aliphatic hydrocarbon at an arbitrary ratio. For example, film formation can be performed as follows.

That is, a silane compound and a fluorine-containing aliphatic hydrocarbon are first supplied to the surface of the metal layer at a mixing ratio of the silane compound ≥ a mixing ratio of the fluorine-containing aliphatic hydrocarbon for adsorption and deposition. Only the silane compound may be used without using a mixed material at the start of the film formation. Subsequently, a film is continuously formed by causing the adsorption and deposition while decreasing (preferably gradually decreasing) the mixing ratio of the silane compound and increasing (preferably gradually increasing) the mixing ratio of the fluorine-containing aliphatic hydrocarbon.

A thickness of the DLC layer 94 is preferably thin from the viewpoint of purpose of the stent 10, and for example, is preferably in a range of from 10 nm to less than 1000 nm, more preferably in a range of from 100 nm to 500 nm, and still more preferably in a range of from 150 nm to 250 nm.

### (Drug-Eluting Coating Layer)

The drug-eluting coating layer 96 is a polymer formed in a layer shape on the surface of the DLC layer 94, and has a function of storing a drug therein. The drug-eluting coating layer 96 has a function of eluting a stored drug when the stent 10 is arranged in a biological organ having a lumen structure such as a blood vessel. A method of storing a drug of the drug-eluting coating layer 96 according to the disclosure is not particularly limited, and a known method can be used. For example, in the drug-eluting coating layer 96 according to the disclosure, a method of storing a drug or a biodegradable polymer containing a drug in a groove or a hole formed in the drug-eluting coating layer 96 may be applied as the method of storing a drug. In addition, in the drug-eluting coating layer 96 according to the disclosure, a method of forming the drug-eluting coating layer 96 with a drug or a biodegradable polymer containing a drug and having biodegradability may be applied as the method of storing a drug. The polymer forming the drug-eluting coating layer 96 may contain a silicone rubber, a urethane rubber, fluororesin, polybutyl acrylate, polybutyl methacrylate, an acrylic rubber, a natural rubber, an ethylene-vinyl acetate copolymer, a styrene-butadiene block copolymer, a styrene-isoprene block copolymer, a styrene-isobutylene block copolymer, or the like. In addition, the biodegradable polymer forming the drug-eluting coating layer 96 may contain a polylactic acid, a poly-β-hydroxybutyric acid, a polyglycolic acid, a lactic acid-glycolic acid copolymer, polycaprolactone, a lactic acid-caprolactone copolymer, a glycolic acid-caprolactone copolymer, a poly-γ-glutamic acid, a glycolic acid-trimethylene carbonate copolymer, or the like.

A thickness of the drug-eluting coating layer 96 is preferably thin from the viewpoint of purpose of the stent 10, and for example, is preferably in a range of from 1 µm to less than 20 µm, and more preferably in a range of from 1 µm to less than 10 µm.

### <Action and Effect>

Next, action and an effect of the disclosure will be described. In this description, in a case in which a configuration or the like similar to a configuration or the like of the stent 10 of the embodiment is used when describing a comparative embodiment with respect to the embodiment, the description is given directly using a reference sign and a name of the configuration or the like.

When the stent 10 is indwelled in a biological organ (hereinafter, a "blood vessel" is used as an example) having a lumen structure such as a blood vessel, the stent 10 in the reduced diameter state is set in an indwelling device such as a delivery catheter. When being ejected from the indwelling device at a predetermined position in the blood vessel, the stent 10 expands in diameter in the blood vessel, comes into close contact with the inner wall of the blood vessel, and is indwelled in the blood vessel. The stent 10 made of the shape memory alloy has favorable followability to deformation of the blood vessel. A drug is eluted from the drug-eluting coating layer 96 of the stent 10 indwelled in the blood vessel.

Here, in the stent 10 of the embodiment, the link 70 connecting the crest 32a of one strut 30 and the crest 34a of the other strut 30 of the two struts 30 adjacent in the axial direction includes the extension portion 44, the extension portion 54, and the bridge portion 72. The stent 10 of the embodiment will be compared with a stent 110 as the comparative embodiment to be described below.

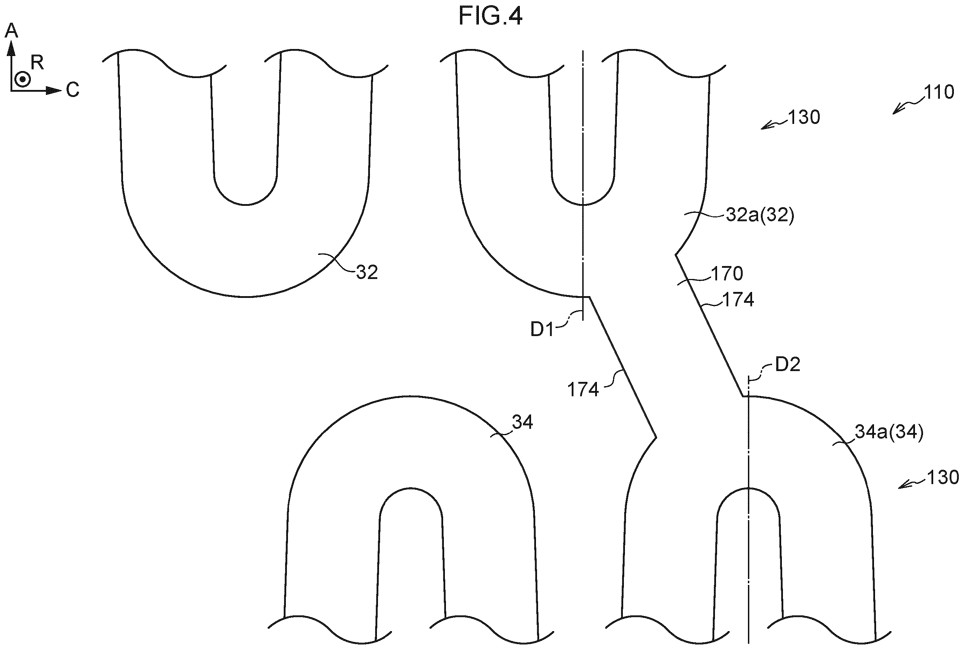

As illustrated in Fig. 4, the stent 110 of the comparative embodiment does not have the extension portions 44 and 54 in the stent 10 of the embodiment. In the stent 110, the crest 32a of one strut 130 and the crest 34a of the other strut 130 of the two struts 130 adjacent in the axial direction are integrally connected by a link 170. The link 170 extends from a portion on a side closer to the crest 34a to be connected with respect to the bisector D1 toward this crest 34a. When viewed conversely, the link 170 extends from a portion on a side closer to the crest 32a to be connected with respect to the bisector D2 toward the crest 32a. That is, in the stent 110 of the comparative embodiment, the link 170 extends from one side with respect to the bisector of each of the crest 32a and the crest 34a (that is, is located only between the bisectors D1 and D2 in the circumferential direction). In the link 170, both ends of an edge portion 174 are linearly formed. That is, in the edge portion 174 of the link 170, one end on the side closer to the crest 32a does not form a common tangent with an edge of the crest 32a at a boundary with respect to the crest 32a when viewed from the radial direction. In addition, in the edge portion 174 of the link 170, the other end on the side closer to the crest 34a does not form a common tangent with an edge of the crest 34a at a boundary with respect to the crest 34a when viewed from the radial direction. Except for the above points, the stent 110 of the comparative embodiment has the same configuration as the stent 10 of the embodiment.

Since the stent 110 of the comparative embodiment has a configuration in which the link 170 extends from one side with respect to the bisector of the crest 32a or the crest 34a, stress is likely to concentrate on the link 170 and a peripheral portion of the link 170 with a transition from one of the reduced diameter state or the expanded diameter state to the other. In particular, in stents having a configuration in which a circumferential position of the crest 32a of one strut 130 is shifted with respect to the crest 34a of the other strut 130, stress is likely to concentrate on the link 170 and the peripheral portion of the link 170 in the stent 110 of the comparative embodiment. There is a possibility that this stress concentration deteriorates shape accuracy of the stent 110 after diameter expansion.

On the other hand, the stent 10 of the embodiment has a configuration in which the extension portion 44 or 54 of one strut 30 extends along the bisector D1 from both the sides of the bisector D1 or D2 bisecting the crest 32a or 34a toward the other strut 30. In the stent 10 of the embodiment, the extension portions 44 and 54 are bridged by the bridge portion 72. As a result, in the stent 10 of the embodiment, stress is less likely to concentrate on the link 70 and a peripheral portion of the link 70 with a transition from the reduced diameter state or the expanded diameter state to the expanded diameter state or the reduced diameter state as compared with the stent 110 of the comparative embodiment. Thus, in stents in which a circumferential position of the crest 32a of one strut 30 is shifted with respect to the crest 34a of the other strut 30, the shape accuracy after diameter expansion of the stent 10 is improved in the stent 10 of the embodiment as compared with the stent 110 of the comparative embodiment.

In addition, the stent 10 of the embodiment has a configuration in which the thickness of the bridge portion 72 is equal to and uniform with the thicknesses of the extension portion 44 and the extension portion 54. As a result, stress is less likely to concentrate on the link 70 and the peripheral portion of the link 70 in the stent 10 of the embodiment as compared with a configuration in which the thickness of the bridge portion 72 is smaller than the thickness of the extension portion 44 or the extension portion 54. Thus, the shape accuracy of the stent 10 is improved in the stent 10 of the embodiment as compared with the configuration in which the thickness of the bridge portion 72 is smaller than the thickness of the extension portion 44 or the extension portion 54.

In addition, the stent 10 of the embodiment has a configuration in which the extension portion 44 has the elliptical shape with the bisector D1 as the major axis, and the extension portion 54 has the elliptical shape with the bisector D2 as the major axis. As a result, stress is less likely to concentrate on the link 70 and the peripheral portion of the link 70 in the stent 10 of the embodiment as compared with a configuration in which the extension portions 44 and 54 extend in the axial direction in an aspect of having a rectangular shape when viewed from the radial direction. Thus, the shape accuracy of the stent 10 is improved in the stent 10 of the embodiment as compared with the configuration in which the extension portions 44 and 54 have the rectangular shape when viewed from the radial direction.

In addition, the stent 10 of the embodiment has a configuration in which one end of the edge portion 74 of the bridge portion 72 on the side closer to the extension portion 44 forms the common tangent with the edge of the extension portion 44 at the boundary with respect to the extension portion 44 when viewed from the radial direction. In addition, the stent 10 of the embodiment has a configuration in which the other end of the edge portion 74 of the bridge portion 72 on the side closer to the extension portion 54 forms the common tangent with the edge of the extension portion 54 at the boundary with respect to the extension portion 54 when viewed from the radial direction. As a result, stress is less likely to concentrate on the link 70 and the peripheral portion of the link 70 in the stent 10 of the embodiment as compared with a configuration in which the edge portion 74 of the bridge portion 72 is linear. Thus, the shape accuracy of the stent 10 is improved in the stent 10 of the embodiment as compared with the configuration in which the edge portion 74 of the bridge portion 72 is linear.

In addition, the stent 10 of the embodiment is made of the shape memory alloy. Thus, in stents made of a shape memory alloy, the shape accuracy of the stent 10 is improved in the stent 10 of the embodiment as compared with the stent 110 of the comparative embodiment.

In addition, the stent 10 of the embodiment has the coating layer 92 on the surface. Since the stent 10 of the embodiment has improved shape accuracy as compared with the stent 110 of the comparative embodiment, it is difficult to distort the coating layer 92. Thus, the coating layer 92 is less likely to be peeled off by distortion of the stent 10 in the stent 10 of the embodiment as compared with the stent 110 of the comparative embodiment.

In the stent 10 of the embodiment, the coating layer 92 includes the DLC layer 94 containing fluorine and silicon. As a result, the DLC layer 94 is less likely to be peeled off by distortion of the stent 10 in the stent 10 of the embodiment as compared with the stent 110 of the comparative embodiment. Thus, a deterioration of the stent 10 over time is suppressed in the stent 10 of the embodiment as compared with the stent 110 of the comparative embodiment.

In the stent 10 of the embodiment, the coating layer 92 includes the drug-eluting coating layer 96. As a result, the drug-eluting coating layer 96 is less likely to be peeled off by distortion of the stent 10 in the stent 10 of the embodiment as compared with the stent 110 of the comparative embodiment. Thus, a drug elution effect of the stent 10 lasts for a long period of time in the stent 10 of the embodiment as compared with the stent 110 of the comparative embodiment.

In addition, in the stent 10 of the embodiment, the thickness T of the strut 30 is in the range of from 0.6 times the minimum width B of the strut 30 to B. Thus, in the stent 10 of the embodiment, the stent 10 has a larger inner diameter as compared with a configuration in which the same outer diameter as the stent 10 having a substantially cylindrical shape is provided and the thickness of the strut 30 exceeds the minimum width B of the strut 30. Therefore, when being indwelled inside a blood vessel, the stent 10 of the embodiment projects less to a lumen of the blood vessel and has less influence on a blood flow of the blood vessel as compared with the configuration in which the same outer diameter as the stent 10 is provided and the thickness of the strut 30 exceeds the minimum width B of the strut 30. As a result, the stent 10 of the embodiment can suppress defective blood circulation in the blood vessel as compared with the configuration in which the same outer diameter as the stent 10 is provided and the thickness of the strut 30 exceeds the minimum width B of the strut 30.

In addition, the stent 10 of the embodiment has a larger thickness as compared with a configuration in which the thickness of the strut 30 is less than 0.6 times the minimum width B of the strut 30, and thus, the stent 10 is less likely to be distorted. Therefore, the shape accuracy of the stent 10 is improved in the stent 10 of the embodiment as compared with the configuration in which the thickness of the strut 30 is less than 0.6 times the minimum width B of the strut 30.

Although a specific embodiment has been described in detail as described above, the disclosure is not limited to the embodiment, and various modifications, changes, and improvements can be made within the scope of the technical idea of the disclosure.

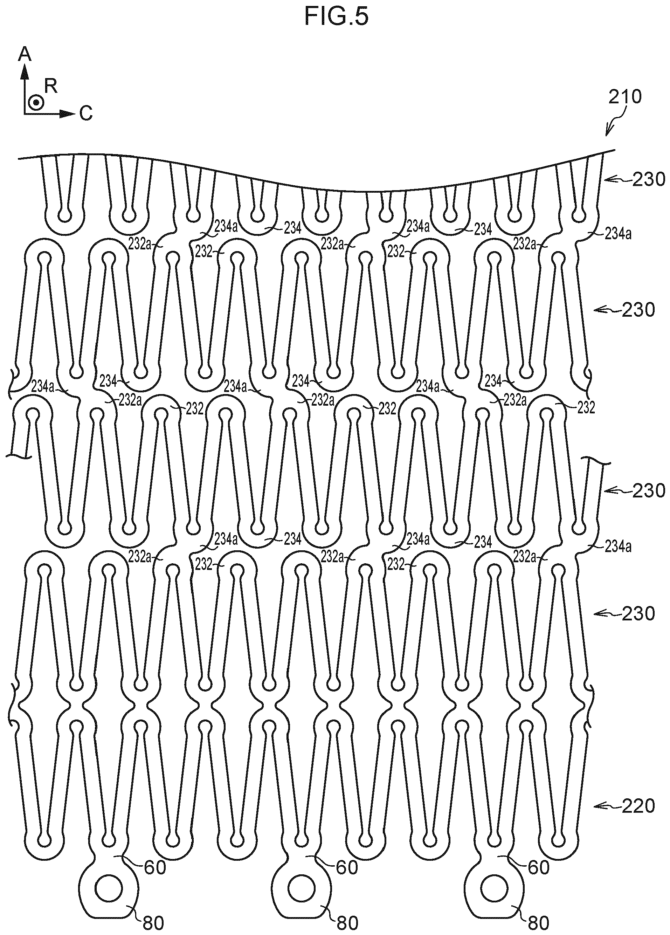

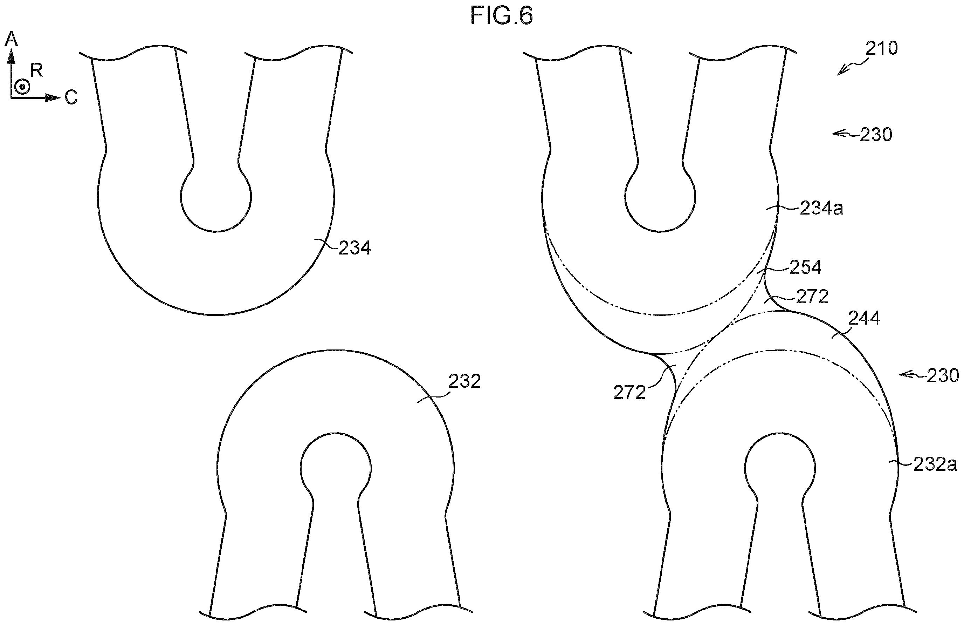

For example, in the stent 10 of the embodiment, the edge of the straight portion 26 of the end strut 20 forms a common tangent of edges of two crests sandwiching the center line CT of the wave of the end strut 20 in the axial direction. However, the straight portion 26 of the end strut 20 according to the disclosure is not limited thereto. For example, as illustrated in Figs. 5 and 6, a straight portion according to the disclosure may be configured to extend along a wavy line of a stent from one end of an arcuate crest 232 or 234 having a central angle exceeding 180° toward a crest on a side opposite to the crest with respect to the center line of the wavy line. Also in this configuration, it is desirable that an interval between a pair of the straight portions 36 connected to one crest 232 or 234 narrows (the angle α is negative) as a distance from the one crest increases in the reduced diameter state.

In addition, the stent 10 of the embodiment has the bridge portion 72 that is formed between the extension portion 44 and the extension portion 54 and has the substantially quadrangular shape when viewed from the radial direction. However, the bridge portion 72 according to the disclosure is not limited thereto. For example, a bridge portion according to the disclosure may have an aspect of being bridged between a first extension portion extending from a first crest and a second extension portion extending from a second crest so as come into contact with each other. Hereinafter, a stent 210, which is an example of a stent that includes the bridge portion having the aspect of being bridged between the first extension portion extending from the first crest and the second extension portion extending from the second crest so as to come into contact with each other will be described.

As illustrated in Fig. 6, the stent 210 has an extension portion 244 extending in an elliptical shape along the axial direction from a crest 232a of one strut 230. In addition, the stent 210 has an extension portion 254 extending in an elliptical shape along the axial direction from a crest 234a of another strut 230 so as to come into contact with the extension portion 244. In addition, the stent 210 has bridge portions 272 bridged between the extension portion 244 and the extension portion 254 on both sides sandwiching a contact portion between the extension portion 244 and the extension portion 254.

In addition, the stent 10 of the embodiment has the coating layer 92 including the DLC layer 94 and the drug-eluting coating layer 96 on the surface. However, the coating layer 92 according to the disclosure may have a configuration including only one of the DLC layer 94 or the drug-eluting coating layer 96. In addition, the stent 10 according to the disclosure may have a configuration in which the coating layer 92 is not provided.

In addition, the extension portions 44 and 54 according to the embodiment have the elliptical shape. However, the extension portions 44 and 54 according to the disclosure may have a shape other than the elliptical shape. For example, the extension portions 44 and 54 according to the disclosure may be formed in a parabolic shape or an isosceles triangular shape. Further, the extension portions 44 and 54 according to the disclosure may be formed in an isosceles trapezoidal shape.

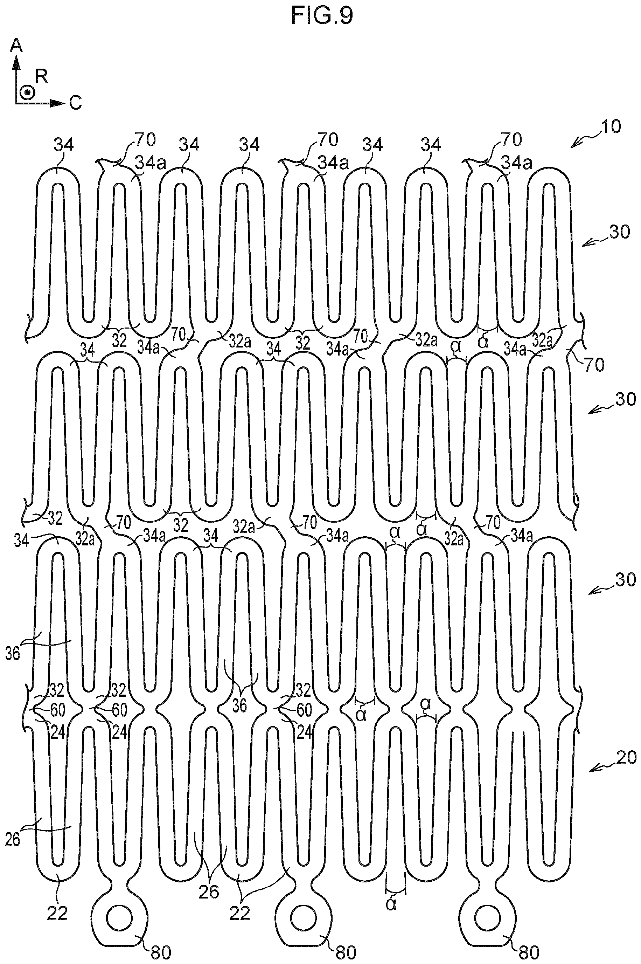

In addition, the plurality of struts 30 according to the embodiment have a configuration in which the two struts 30 adjacent to each other in the axial direction are arranged such that the phases of the waves in the circumferential direction are shifted. However, as illustrated in Fig. 9, the plurality of struts 30 according to the disclosure may be arranged side by side in the axial direction such that phases of waves in the circumferential directions of the respective struts 30 are the same as each other. In this case, the crest 32a and the crest 34a connected by the link 70 are located to be shifted by half a wavelength of the wave in two adjacent struts 30, and one of the crests protrudes toward the other.

In addition, each of the struts 30 according to the embodiment has nine crests 32 and nine crests 34. However, the number of crests of a strut of a stent according to the disclosure is not particularly limited, and can be set in accordance with a size of a biological organ having a lumen structure in which the stent is to be indwelled.

In addition, two adjacent struts 30 according to the embodiment are connected by three links 70 arranged at equal intervals in the circumferential direction. However, the arrangement and the number of links according to the disclosure are not particularly limited, and can be set in accordance with a size of a biological organ having a lumen structure in which a stent is to be indwelled.

## Claims

1. A stent, comprising:
a first strut that is arranged along a cylindrical surface and forms a wave shape having an amplitude direction that is an axial direction of the cylindrical surface;
a second strut that is arranged along the cylindrical surface, that forms a wave shape having an amplitude direction that is the axial direction, and that is adjacent to the first strut in the axial direction;
a first extension portion that is formed on a first crest and extends along a first bisector, bisecting the first crest, from both sides with respect to the first bisector toward the second strut, the first crest being at least one of a plurality of crests in the first strut protruding toward the second strut;
a second extension portion that is formed on a second crest and extends along a second bisector, bisecting the second crest, from both sides with respect to the second bisector toward the first strut, the second crest being displaced in a circumferential direction with respect to the first crest among a plurality of crests in the second strut protruding toward the first strut side; and
a bridge portion extending from the first extension portion to the second extension portion.

2. The stent according to claim 1, wherein a thickness of the bridge portion is equal to and uniform with a thickness of the first extension portion or the second extension portion.

3. The stent according to claim 1 or 2, wherein:
the first extension portion has an elliptical shape whose major axis is the first bisector, and
the second extension portion has an elliptical shape whose major axis is the second bisector.

4. The stent according to any one of claims 1 to 3, wherein, when viewed along a radial direction of the cylindrical surface, one end of an edge portion of the bridge portion on a side closer to the first extension portion has a curved shape forming a common tangent with an edge of the first extension portion at a boundary with respect to the first extension portion, and another end of the edge portion of the bridge portion on a side closer to the second extension portion has a curved shape forming a common tangent with an edge of the second extension portion at a boundary with respect to the second extension portion.

5. The stent according to any one of claims 1 to 4, comprising a shape memory alloy.

6. The stent according to any one of claims 1 to 5, further comprising a coating layer on a surface of the stent.

7. The stent according to claim 6, wherein the coating layer comprises a ceramic layer.

8. The stent according to claim 6 or 7, wherein the coating layer comprises a drug-eluting coating layer.

9. The stent according to any one of claims 1 to 8, wherein a thickness of each of the first strut and the second strut is in a range of from 0.6 times a minimum width B of each of the first strut and the second strut in a direction along the cylindrical surface, to the minimum width B.
